(19) Europäisches Patentamt
European Patent Office
Office européen des brevets

(11) Publication number: **0 155 006**
**B1**

(12) # EUROPEAN PATENT SPECIFICATION

(45) Date of publication of patent specification: **24.10.90**

(51) Int. Cl.$^5$: **C 07 D 215/56,** A 61 K 31/47

(21) Application number: **85103012.2**

(22) Date of filing: **15.03.85**

(30) Priority: **16.03.84 IT 2007584**

(43) Date of publication of application:
**18.09.85 Bulletin 85/38**

(45) Publication of the grant of the patent:
**24.10.90 Bulletin 90/43**

(84) Designated Contracting States:
**AT BE CH DE FR GB IT LI LU NL SE**

(56) References cited:
**EP-A-0 067 666**
**EP-A-0 117 473**
**FR-A-2 437 406**

**The file contains technical information submitted after the application was filed and not included in this specification**

(73) Proprietor: **FARMADES S.p.A.**
**Via Tor Cervara, 282**
**I-00100 Rome (IT)**

(72) Inventor: **Di Schiena, Michele**
**Via Brunelleschi, 30/C**
**I-20090 Trezzano Sul Naviglio Milan (IT)**

(74) Representative: **Bianchetti, Giuseppe**
**Studio Consulenza Brevettuale Via Rossini, 8**
**I-20122 Milan (IT)**

**EP 0 155 006 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention refers to compounds of general formula I

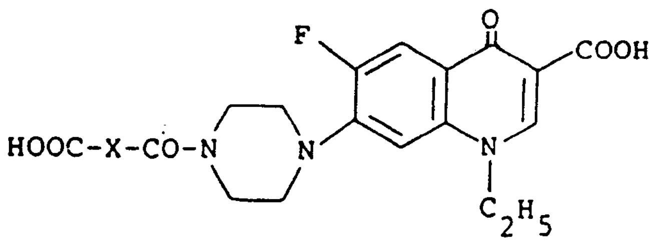

(I)

wherein the group HOOC—X—CO— is a residue of a dicarboxylic acid selected in the group of malonic, succinic, malic, maleic, fumaric, glutaric, adipic, cyclohexan-1,4-dicarboxylic, camphoric, endo-bicyclo[2.2.2]oct-5-en-2,3-dicarboxylic, phthalic, terephthalic, isophthalic and their salts with pharmaceutically acceptable metals or organic bases.

The invention concerns also possible hydrated forms and possible enantiomers and/or diastereoisomers of compounds (I) and salts thereof.

Examples of salts of compounds I are the salts with the following inorganic pharmaceutically acceptable bases: lithium, sodium, potassium, rubidium, cesium, ammonium, calcium, magnesium, aluminium, iron, zinc, manganese, silver, gold, platinum, triethylamine, tripropylamine, 2-dimethylaminoethanol, 2-diethylaminoethanol, mono, di- and tri-ethanolamine, morpholine, hydroxyethylpyrrolidine, tromethanol, urothopine, choline, lysine, arginine, histidine, coffeine, theobromine, procaine, piperidine, N-ethylpiperidine, hydrabamine, purine, piperazine, polyamine resins, and the salification may involve one or both carboxy groups.

Compounds (I) can be considered derivatives of 1-ethyl-6-fluoro-1,4-dihydro-4-oxo-7-(1-piperazinyl)-quinoline-3-carboxylic acid disclosed in FR—A—2437406, known (with the name "norfloxacine") for its antibacterial activity, particularly used for the treatment of urinary and genito-urinary tract infections (cystitis, pyelitis, cystopyelitis, pyelonephrites, gonhorrea).

A remarkable drawback of 1-ethyl-6-fluoro-1,4-dihydro-4-oxo-7-(1-piperazinyl)-quinoline-3-carboxylic acid consists in that it is practically insoluble in water; also its salts both with acids and with organic or inorganic bases are practically insoluble in water. The only salts relatively soluble give anyway aqueous solutions whose pH is not physiologically acceptable.

This feature limits the therapeutic use of norfloxacine, as the injectable administration forms and the oral soluble administration forms such as syrups, bottles or monodose vials, the compositions in form of drops, are excluded: especially the latter ones are particularly important since they are suited to a remarkable flexibility of the therapeutic dosage. EP—A—67666 discloses salts of norfloxacine and derivatives with galacturonic aspartic, gluconic or glutonic acid, which, being more hydrosoluble, are suited for the parenteral administration. EP—A—117473, published after the priority date of the present application, discloses derivative of cyprofloxacine (an analogue of norfloxacine having a 1-cyclopropyl group instead of a 1-ethyl groupo) wherein the piperazinyl group may be substituted by an acyl group in position 4.

On the other side, salts of compounds (I) providing the object of the present invention, exhibit the particular characteristics of being remarkably soluble in water and of giving aqueous solutions with pH ranging in physiological values, with toxicity and antibacterial activity characteristics practically unchanged in comparison with 1-ethyl-6-fluoro-1,4-dihydro-4-oxo-7-(1-piperazinyl)-quinoline-3-carboxylic acid.

A further object of the invention is therefore provided by pharmaceutical forms for human or veterinary use, liquid or lyophilized, containing as active principle the compounds (I) and their salts, such as syrups, bottles, monodose vials, drops forms (including extemporary forms). In these pharmaceutical forms the new compounds can be administered fitting the posology according to the therapeutic case, to the clinical seriousness, to the patient's age, to the need of carrying out an intensive or maintenance therapy, obtaining thus the particular utility of the flexibility of the therapeutic dosage.

An admimistration form particularly useful in the therapy of pathological forms, containing the new compounds as active principles, is the injectable form, by this fact it is in fact possible to carry out timely intensive therapies, obtaining high drug blood level as well as providing the drug administration whenever the oral route turns out to be not advisable or not enough sure for a suitable drug use. Of course, also the other pharmaceutical forms known in the art can conveniently use the new compound. Among the latter ones, there are included also the pharmaceutical forms for topical use such as powders, creams, vaginal and urethral washings.

The preparation of the compounds according to the present invention is based on techniques already known in the art, substantially consisting in reacting 1-ethyl-6-fluoro-1,4-dihydro-4-oxo-7-(1-piperazinyl)-quinoline-3-carboxylic acid with a suitable N-acylating derivative of the above mentioned bicarboxylic acids, such as for instance anhydrides or acyl monochlorides.

The reaction solvent and the condition thereof obviously depend on the type of the N-acylating agent used and are anyway known to the skilled in the art.

By using for instance an anhydride, the reaction can be carried out using as a solvent methylene chloride, chloroform, glacial acetic acid, pyridine, the latter even in non anhydrous form, for instance with 5—90% of water. The anhydride can be used in the stoichiometric ratio or in excess up to 100%, but preferably in 20% excess.

Compounds I are obtained in the acid form and are isolated as such with the common techniques known in the art, as for instance by filtration.

The so obtained acid is salified by means of suitable salifying agents, for instance alkali or alkali-earth metal hydroxides, carbonates or bicarbonates, or organic bases of the above mentioned kind.

The salification techniques are those already known in the art and consists for instance of reacting the acid compound with the salifying agent in a stoichiometric ratio. The salification can be conveniently and suitably carried out in water or in suitable anhydrous or variously hydrated solvents such as methanol, ethanol, isopropanol, acetone.

The compounds according to the invention are therefore isolated by means of the techniques known in the art, which essentially consist in evaporating the solvent or in the precipitation. If the salification is carried out in water the solution can be used as such for instance in the preparation of the injectable forms after suitable sterilization and depyrogenation.

The following examples further illustrate the present invention.

### Example 1

1-Ethyl-6-fluoro-1,4-dihydro-4-oxo-7-[4-(3-carboxypropionyl)-1-piperazinyl]-quinolin-3-carboxylic acid

3.19 Grams of 1-ethyl-6-fluoro-1,4-dihydro-4-oxo-7-(1-piperazinyl)-quinoline-3-carboxylic acid were dissolved in 120 ml of methylene chloride (free from alcohols). 1 Gram of finely ground succinic anhydride was added and the mixture was left to react for about 30′ under vigorous stirring. The mixture was then refluxed for 5—10 minutes, then cooled to room temperature, always under vigorous stirring, in order to transform the semisolid precipitate initially formed into a crystalline solid which was pump filtered and repeatedly washed on the filter with methylene chloride. After drying at 40°C, 2.7 g of colourless crystalline product, melting at 242—246°C, were obtained.

Analysis: for $C_{20}H_{22}FN_3O6$ (MW = 419.39)

| | | | |
|---|---|---|---|
| cal. % | C = 57.23; | B = 4.25; | N = 10.01 |
| found % | C = 57.01; | H = 5.33; | N = 9.89. |

The structure of the compound was also confirmed from the NMR spectrum, carried out in hexadeutero-dimethylsulfoxide (rif. TSP).

| | | |
|---|---|---|
| δ, ppm: | 1.46 | (3H, $CH_3$); |
| | : 2.51 | (4H, $CO—CH_2CH_2—CO$); |
| | : 3.37—3.72 | (8H, piperazine); |
| | : 4.55 | (2H, $CH_3—$*$CH_2$*$—N$); |
| | : 7.16 | (1H, =CH—N); |
| | : 7.74 | (1H, =CH—(5)); |
| | : 7.84 | (1H, =CH—(8)). |

### Example 2

1-Ethyl-6-fluoro-1,4-dihydro-4-oxo-7-[4-(3-carboxypropionyl)-1-piperazinyl]-quinolin-3-carboxylic acid

1.2 Grams of succinic anhydride was added to the solution of 3.19 g of 1-ethyl-6-fluoro-1,4-dihydro-4-oxo-7-(1-piperazinyl)-quinoline-3-carboxylic acid, under stirring. The separation of a crystalline product started almost immediately. After stirring overnight, the precipitate was pump filtered, washed with 5 × 5 ml of acetic acid and dryed under vacuum at 40°C. 3 Grams of compound melting at 244—246°C, identical to that obtained according to the Example 1, were obtained.

The same result, with slightly lower yields, was achieved by carrying out the reaction in 50 ml of 10% acetic acid in water (v/v), and using 2 g (instead of 1.2 g) of succinic anhydride, or in 50 ml of 10% pyridine in water (v/v) and 2 g of succinic anhydride.

### Example 3

Di-sodium salt of 1-ethyl-6-fluoro-1,4-dihydro-4-oxo-7-[4-(3-carboxypropionyl)-1-piperazinyl]-quinolin-3-carboxylic acid

0.84 Grams (0.01 moles) of $NaHCO_3$ were added, under stirring, to a suspension of 2 g (0.005 moles) of the compound obtained according to the Examples 1—2 in 8 ml of water. A perfectly clear solution was obtained, with effervescence, having pH 7.5. The addition of 100 ml of absolute ethanol caused the precipitation of a crystalline solid which was pump filtered and washed with little 95% ethanol and then with acetone.

1.5 Grams of the desired salt, which swells without melting at 245°C, were obtained.

Example 4

Di-sodium salt of 1-ethyl-6-fluoro-1,4-dihydro-4-oxo-7-[4-(3-carboxypropionyl)-1-piperazinyl]-quinolin-3-carboxylic acid

5 Ml of 2N NaOH were added to a stirred suspension of 2 g (0.005 moles) of 1-ethyl-6-fluoro-1,4-dihydro-4-oxo-7-[4-(3-carboxypropionyl)-1-piperazinyl]-quinolin-3-carboxylic acid in 10 ml of water. After stirring for 15 hours, the mixture was evaporated to dryness under vacuo. The same salt of Example 3, which was thoroughly washed with acetone, was obtained.

Example 5

Salts of 1-ethyl-6-fluoro-1,4-dihydro-4-oxo-7-[4-(3-carboxypropionyl)-1-piperazinyl]-quinolin-3-carboxylic acid

Operating as described in Example 4, with an equivalent amount of the potassium, lithium, ammonium, calcium hydroxides or with equivalent amounts of triethylamine, N,N-diethylaminoethanol, tromethamol, choline, lysine, arginine, the corresponding salts were obtained.

Example 6

Aluminium salt of 1-ethyl-6-fluoro-1,4-dihydro-4-oxo-7-[4-(3-carboxypropionyl)-1-piperazinyl]-quinolin-3-carboxylic acid

A solution of 6.6 g of aluminium phosphate and 10 g of sodium acetate in 200 ml of water was added to the solution of 13.8 g of di-sodium salt, prepared according to Examples 3 or 4, in 200 ml of water. After stirring for 15 hours the solid formed was pump filtered, washed several times with water and dryed at 40°C. 12 Grams of the desired salt were obtained.

Example 7

1-Ethyl-6-fluoro-1,4-dihydro-4-oxo-7-[4-(3-carboxy-*cis*-propenoyl)-1-piperazinyl]-quinolin-3-carboxylic acid

The procedure was the same as that described in the Example 1, substituting succinic anhydride with 0.98 g of maleic anhydride and leaving under stirring for 48 hours. 2.8 Grams of crystalline, colourless product, m.p. 221—224°C, were obtained.

Analysis: for $C_{20}H_{20}FN_3O_6$ (MW 417.38)

| | | | |
|---|---|---|---|
| calc. % | C = 57.50; | H = 4.81; | N = 10.06 |
| found % | C = 57.38; | H = 4.84; | N = 9.94. |

Example 8

Monoamide of camphoric acid with 1-ethyl-6-fluoro-1,4-dihydro-4-oxo-7-(piperazinyl]-quinolin-3-carboxylic acid

The same procedure as in the Example 1 was followed, but substituting succinic anhydride with 1.8 g of camphoric anhydride. 2.32 Grams of the desired compound were obtained.

Example 9

Monoamide of phthalic acid with 1-ethyl-6-fluoro-1,4-dihydro-4-oxo-7-(piperazinyl)-quinolin-3-carboxylic acid

The same procedure as in the Example 1 was followed, using however 1.48 g of phthalic anhydride. 3.1 Grams of the desired compound were obtained.

Example 10

Monoamide of endo-bicyclo[2.2.2]-oct-5-en-2,3-dicarboxylic acid with 1-ethyl-6-fluoro-1,4-dihydro-4-oxo-7-(piperazinyl]-quinolin-3-carboxylic acid

The same procedure described in the Example 1 was followed, using however 1.78 g of the anhydride of endo-bicyclo[2.2.2]-oct-5-en-2,3-dicarboxylic acid instead of succinic anhydride. 2.8 Grams of the desired compound were obtained.

As already stated, the present invention also refers to all the industrial aspects connected with the use of compounds of formula I and salts thereof as antibacterial agents. An essential aspect of the invention is therefore provided by pharmaceutical compositions containing predetermined amounts of one or more compounds of formula (I), or of the corresponding salts, together with carriers, excipients, conservative agents commonly used in pharmaceutical practice. Examples of such compositions are hereinafter given, referring to the case — also purely illustrative — of the di-sodium salt of 1-ethyl-6-fluoro-1,4-dihydro-4-oxo-7-[4-(3-carboxypropionyl)-1-(piperazinyl]-quinolin-3-carboxylic acid, hereinafter defined — for the sake of shortness — with the abbreviation "sodium IVM/14" or of the acid as such ("acid IVM/14").

Example 11

Powders for showers irrigations, vaginal lavages and for urethral irrigations

| | |
|---|---|
| IVM/14 Sodium | g 1 |
| Borax | g 1 |
| Sodium chloride | g 8 |
| Hydrosoluble chlorophylla | g 0.05 |

To be dissolved in 1—2 l of water depending on the use.

Example 12

Vaginal ovules

| | |
|---|---|
| IVM/14 Sodium | g 0.015 |
| Polyglycole q.s. to | g 1 |

Example 13

Composition for veterinary use

| | |
|---|---|
| IVM/14 Sodium | g 200 |
| Glycerin | g 200 |
| Purified water q.s. to | g 1000 |

In the infections of the uro-genital tracts: lavages with 0.5—5% solutions.

Example 14

Bottles and phials for oral route

Each bottle or phials contains:

| | |
|---|---|
| IVM/14 Sodium | g 0.580 |
| Sorbitole | g 3 |
| Raspberry flavour | g 0.001 |
| Distilled water q.s. to | ml 10 |

Example 15

Syrup

| | |
|---|---|
| IVM/14 Sodium | g 5 |
| Saccharose | g 20 |
| Methyl paraoxybenzoate | g 0.08 |
| Propyl paraoxybenaozte | g 0.02 |
| Caramel | ml 0.01 |
| Apricot flavour | ml 0.01 |
| Distilled water q.s. to | ml 100 |

Example 16

Oral drops

| | |
|---|---|
| IVM/Sodium | g 40 |
| Sorbitol 70% solution | ml 50 |
| Distilled water q.s. to | ml 100 |

Example 17

Granulate and effervescent tablets

Each tablets or sachet of effervescent granulate contains:

| | |
|---|---|
| IVM/14 | mg 525 |
| Sodium bicarbonate | mg 276 |
| Sodium citrate | mg 250 |
| Sodium saccharine | mg 1.5 |
| Lemon flavour | mg 4 |
| Mannitol | mg 318 |
| Polyvinylpyrrolidone | mg 1 |

Example 18

Oral capsules

| | |
|---|---|
| IVM/14 Acid | mg 525 |
| Lactose | mg 75 |

Example 19

Oral tablets

| | |
|---|---|
| IVM/14 Acid | mg 525 |
| Lactose | mg 25 |
| Maize starch | mg 10 |

Example 20

Injectable preparation

525 Grams of IVM/14 acid were suspended in 4000 ml of distilled water. Sodium bicarbonate (276 g) was carefully added under stirring. The obtained solution with pH 7.2—7.4 was filtered through sterilizing and depyrogenating membrane. The so treated solution was brought to the exact volume of 5000 ml with sterile apyrogenic water.

By this solution, there were prepared:

a) Lyophilized injectable bottles

Bottles for injectable use each containing 5 ml of solution, corresponding to 0.525 g of IVM/14 acid, were prepared, by the usual methods, starting from the above prepared solution.

The bottles so filled are lyophilized and stored together with a solvent phial comprising 5 ml of sterile-apyrogenic water.

b) Injectable phials

Phials, each containing 2.5 ml of solution, corresponding to 0.262 g of IVM/14 acid, are prepared, by the usual methods, starting from the above prepared solutions.

**Claims for the Contracting States: BE CH DE FR GB IT LI LU NL SE**

1. Compounds of the general formula I

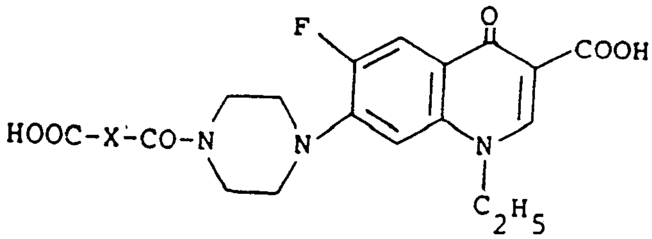

(I)

wherein the group HOOC—X—CO is a residue of a dicarboxylic acid selected in the group of
malonic acid;
succinic acid;
glutaric acid;
adipic acid;
maleic acid;
fumaric acid;
malic acid;
camphoric acid;
endo-bicyclo[2.2.2]-oct-5-en-2,3-dicarboxylic acid;
cyclohexan-1,4-dicarboxylic acid;
phthalic acid;
isophthalic acid;
terephthalic acid,

and their salts with pharmaceutically acceptable metals or organic bases, possible hydrated forms and enantiomers and/or diastereoisomers.

2. Salts of compound (I) with lithium, sodium, potassium, rubidium, cesium, ammonium, calcium, magnesium, aluminium, iron, zinc, manganese, silver, gold, platinum, triethylamine, tripropylamine, 2-dimethylaminoethanol, 2-diethylaminoethanol, mono, di- and tri-ethanolamine, morpholine, hydroxyethylpyrrolidine, tromethanol, urothopine, choline, lysine, arginine, histidine, coffeine, theobromine, procaine, piperidine, N-ethylpiperidine, hydrabamine, purine, piperazine, polyamine resins, and the salification may involve one or both carboxy groups.

3. The di-sodium salt of 1-ethyl-6-fluoro-1,4-dihydro-4-oxo-7-[4-(3-carboxy-propionyl)-1-piperazinyl]-quinolin-3-carboxylic acid.

4. Process for the preparation of compounds of claims 1—3 characterized in that 1-ethyl-6-fluoro-1,4-dihydro-4-oxo-7-(1-piperazinyl)-quinoline-3-carboxylic acid is reacted with a N-acylating derivative of the dicarboxylic acids as defined in claim 1 having formula HOOC—X—COOH, and that the compounds so obtained are optionally salified by known methods.

5. Process according to claim 4 characterized in that monoacyl chlorides or anhydrides are used as N-acylating derivative of the dicarboxylic acid of formula HOOC—X—COOH.

6. Pharmaceutical compositions having antibacterial activities, for human or veterinary use, characterized by containing as active principle an effective amount of at least one compound of claims 1—3 in admixture with at least one inert carrier or excipient.

7. Pharmaceutical compositions according to claim 6 for oral, parenteral or topical administration in form of bottles, vials, syrups, drops, effervescent granulate or effervescent tablets, capsule, tablets, injectable sterile vials or bottles, powder for washings, irrigations, vaginal and urethral lavages, vaginal ovules.

**Claims for the Contracting State: AT**

1. Process for the preparation of compounds having formula I

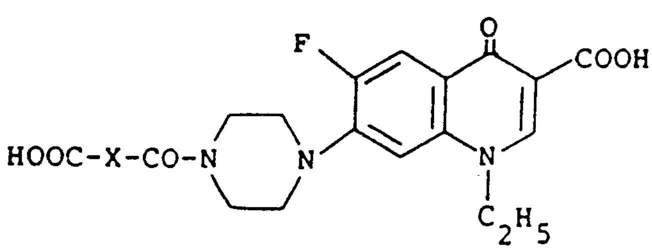

(I)

wherein the group HOOC—X—CO— is a residue of a dicarboxylic acid selected in the group of malonic, succinic, malic, maleic, fumaric, glutaric, adipic, cyclohexan-1,4-dicarboxylic, camphoric, endo-bicyclo[2.2.2]oct-5-en-2,3-dicarboxylic, phthalic, terephthalic, isophthalic, and their salts with pharmaceutically acceptable metals or organic bases, possible hydrated forms and enantiomers and/or diastereoisomers, characterized in that 1-ethyl-6-fluoro-1,4-dihydro-4-oxo-7-(1-piperazinyl)-quinoline-3-carboxylic acid is reacted with a N-acylating derivative of the dicaboxylic acids as defined above having formula HOOC—X—COOH, and that the compounds so obtained are optionally salified by known methods.

2. Process according to claim 1 characterized in that monoacyl chlorides or anhydrides are used as N-acylating derivative of the dicarboxylic acid of formula HOOC—X—COOH.

**Patentansprüche für die Vertragsstaaten: BE CH DE FR GB IT LI LU NL SE**

1. Verbindungen der allgemeinen Formel I

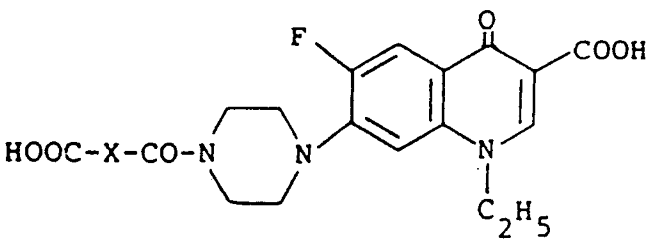

(I)

dadurch gekennzeichnet, daß die Gruppe HOOC—X—CO ein Rest einer Dicarbonsäure aus der Gruppe von
Malonsäure,
Bernsteinsäure,
Glutarsäure,
Adipinsäure,
Maleinsäure,
Fumarsäure,
Äpfelsäure,
Kampfersäure,
Endo-bicyclo[2.2.2]-oct-5-en-2,3-carbonsäure,
Cyclohexan-1,4-dicarbonsäure,
Phthalsäure,
Isophthalsäure und
Terephthalsäure ist,
und deren Salze mit pharmazeutisch unbedenklichen Metallen oder organischen Basen, gegebenenfalls in Form von Hydraten und Enantiomere und/oder Diastereoisomere.

2. Salze der Verbindungen gemäß Anspruch 1 mit Lithium, Natrium, Kalium, Rubidium, Cäsium, Ammonium, Calcium, Magnesium, Aluminium, Eisen, Zink, Mangan, Silber, Gold, Platin, Triethylamin, Tripropylamin, 2-Dimethylaminoethanol, 2-Diethylaminoethanol, Mono-, Dip und Triethanolamin, Morpholin, Hydroxyethylpyrrolidin, Tromethamol, Urotropin, Cholin, Lysin, Arginin, Histidin, Coffein, Theobromin, Procain, Piperidin, N-Ethylpiperidin, Hydrabamin, Purin, Piperazin, Polyaminharze, wobei die Salzbildung sich auf eine oder beide Carboxylgruppen erstrecken kann.

3. Dinatriumsalz von 1-Ethyl-6-fluor-1,4-dihydro-4-oxo-7-[4-(3-carboxy-propionyl)-1-piperazinyl]-chinolin-3-carbonsäure.

4. Verfahren zur Herstellung von Verbindungen nach Anspruch 1 bis 3, dadurch gekennzeichnet, daß 1-Ethyl-6-fluor-1,4-dihydro-4-oxo-7-(1-piperazinyl)-chinolin-3-carbonsäure mit einem N-acylierenden Derivat der in Anspruch 1 angegebenen Carbonsäuren der Formel HOOC—X—COOH umgesetzt wird und die so erhaltenen Verbindungen gegebenenfalls nach bekannten Verfahren in Salze überführt werden.

5. Verfahren nach Anspruch 4, dadurch gekennzeichnet, daß die Monoacylchloride oder -anhydride als N-acylierendes Derivat der Dicarbonsäure der allgemeinen Formel HOOC—X—COOH verwendet werden.

6. Pharmazeutische Zusammensetzungen mit antibakterieller Wirkung zur Verwendung in der Human- und Veterinärmedizin, dadurch gekennzeichnet, daß sie als Wirkstoff eine wirksame Menge mindestens einer Verbindung nach Anspruch 1 bis 3 im Gemisch mit mindestens einem inerten Träger oder Excipienten aufweisen.

7. Pharmazeutische Zusammensetzungen nach Anspruch 6 zur oralen, parenteralen oder äußerlichen Verabfolgung in Form von Flaschen, Ampullen, Sirupen, Tropfen, Brausegranulat, oder Brausetabletten, Kapseln, Tabletten, injizierbaren sterilen Ampullen oder Flaschen, Pulven für Wäschen, Spülungen, vaginale und Harnröhrenspülungen, Vaginalkugeln.

**Patentansprüche für den Vertragsstaat: AT**

1. Verfahren zur Herstellung von Verbindungen der allgemeinen Formel I

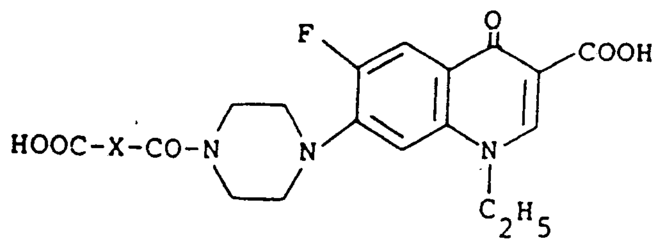

(I)

worin die Gruppe HOOC—X—CO ein Rest einer Dicarbonsäure aus der Gruppe Malonsäure, Bersteinsäure, Äpfelsäure, Maleinsäure, Fumarsäure, Glutarsäure, Adipinsäure, Cyclohexan-1,4-dicarbonsäure, Kampfersäure, Endo-bicyclo-[2.2.2]-oct-5-en-2,3-dicarbonsäure, Phthalsäure, Terephthalsäure und Isophthalsäure ist, und deren Salzen mit pharmazeutisch unbedenklichen Metallen oder organischen Basen, gegebenenfalls in Form von Hydraten und Enantiomeren und/oder Diasteroisomeren, dadurch gekennzeichnet, daß 1-Ethyl-6-fluor-1,4-dihydro-4-oxo-7-(1-piperazinyl)-chinolin-3-carbonsäure mit einem N-acylierenden Derivat der oben definierten Dicarbonsäuren der Formel HOOC—X—COOH umgesetzt wird und die so erhaltenen Verbindungen gegebenenfalls nach bekannten Verfahren in Salze überführt werden.

2. Verfahren nach Anspruch 1, dadurch gekennzeichnet, daß Monoacylchloride oder -anhydride als N-acylierendes Derivat der Dicarbonsäure der Formel HOOC—X—COOH verwendet werden.

**Revendications pour les Etats contractants: BE CH DE FR GB IT LI LU NL SE**

1. Composés de la formule générale I

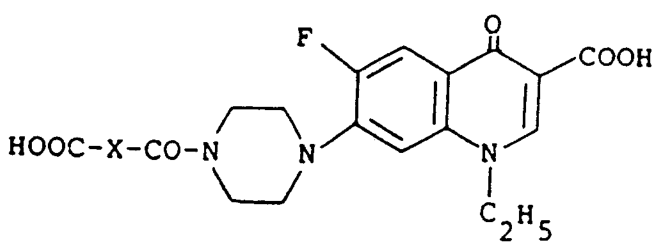

(I)

dans laquelle le groupe HOOC—X—CO— est un reste d'un acide dicarboxylique choisi dans le groupe formé par
- l'acide malonique;
- l'acide succinique;
- l'acide glutarique;
- l'acide adipique;
- l'acide maléique;
- l'acide fumarique;
- l'acide malique;
- l'acide camphorique;
- l'acide endo-bicyclo[2.2.2]oct-5-ène-2,3-dicarboxylique;
- l'acide cyclohexane-1,4-dicarboxylique;
- l'acide phtalique;
- l'acide isophtalique;
- l'acide téréphtalique,

et leurs sel formés avec des métaux ou des bases organiques pharmaceutiquement acceptables, leurs formes hydratées possibles et des énantiomères et/ou diastéréoisomères.

2. Sels de composé (I) formés avec le lithium, le sodium, le potassium, le rubidium, le césium, l'ammonium, le calcium, le magnésium, l'aluminium, le fer, le zinc, le manganèse, l'argent, l'or, le platine, la triéthylamine, la tripropylamine, le 2-diméthylaminoéthanol, le 2-diéthylaminoéthanol, les mono-, di- et triéthanolamines, la morpholine, l'hydroxyéthylpyrrolidine, le trometamol, l'urotropine, la cholne, la lysine, l'arginine, l'histidine, la caféine, la théobromine, la procaïne, la pipéridine, la N-éthylpipéridine, l'hydrabamine, la purine, la pipérzine, des résines polyaminées, et la salification peut impliquer un seul des groupes carboxyle ou les deux.

3. Le sel disodique de l'acide 1-éthyl-6-fluoro-1,4-dihydro-4-oxo-7-[4-(3-carboxypropionyl)-1-pipérazinyl]quinoléine-3-carboxylique.

4. Procédé pour la préparation de composés des revendications 1 à 3, caractérisé en ce que l'acide 1-éthyl-6-fluoro-1,4-dihydro-4-oxo-7-(1-pipérazinyl)quinoléine-3-carboxylique est amené à réagir avec un dérivé N-acylant des acides dicarboxyliques tels que définis dans la revendication 1 répondant à la formule HOOC—X—COOH, et en ce que les composés ainsi obtenus sont facultativement salifiés par des méthodes connues.

5, Procédé selon la revendication 4, caractérisé en ce que des monochlorures ou mono-anhydrides d'acyle sont utilisés à titre du dérivé N-acylant de l'acide dicarboxylique de formule HOOC—X—COOH.

6. Compositions pharmaceutiques selon d'activités antibactériennes, pour usage en médecine humaine ou vétérinaire, caractérisées en ce qu'elles contiennent, comme principe actif, une quantité efficace d'au moins un composé des revendications 1 à 3 en mélange avec au moins un support ou excipient inerte.

7. Compositions pharmaceutiques selon la revendication 6 destinées à l'administration orale, parentérale ou topique, sous forme de flacons, ampoules, sirops, gouttes, granulés effervescents ou comprimés effervescents, gélules ou capsules, comprimés, ampoules ou flacons stériles pour injections, poudre pour lavages, irrigations, lavages vaginaux ou urétraux, ou ovules vaginaux.

**Revendications pour l'Etat contractant: AT**

1. Procédé pour la préparation de composés répondant à la formule I

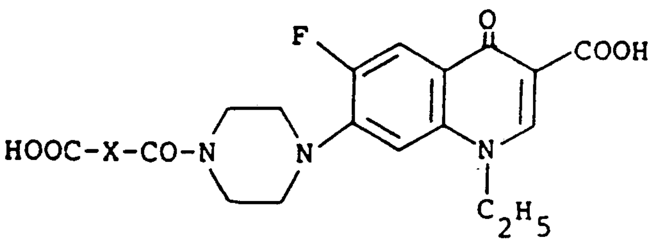

(I)

dans laquelle le groupe HOOC—X—CO— est un reste d'un acide dicarboxylique choisi dans le groupe formé par les acides malonique, succinique, malique, maléique, fumarique, glutarique, adipique, cyclohexane-1,4-dicarboxylique, camphorique, endo-bicyclo[2.2.2]oct-5-ène-2,3-dicarboxylique, phtalique, téréphtalique et isophtalique, et leurs sels formés avec des métaux ou des bases organiques pharmaceutiquement acceptables, leurs formes hydratées possibles et des énantiomères et/ou diastéréoisomères, caractérisé en ce que l'acide 1-éthyl-6-fluoro-1,4-dihydro-4-oxo-7-(1-pipérazinyl)quinoléine-3-carboxylique est amené à réagir avec un dérivé N-acylant des acides dicarboxyliques tels que définis ci-dessus répondant à la formule HOOC—X—COOH, et en ce que les composés ainsi obtenus sont facultativement salifiés par des méthodes connues.

2. Procédé selon la revendication 1, caractérisé en ce que des monochlorures ou mono-anhydrides d'acyle sont utilisés à titre du dérivé N-acylant de l'acide dicarboxylique de formule HOOC—X—COOH.